# EUROPEAN PATENT APPLICATION

(11) **EP 4 717 231 A1**
(43) Date of publication of application: **01.04.2026**
(21) Application number: 24810274.1
(22) Date of filing: 15.05.2024
(51) Int. Cl.: A61F 2/24, A61M 25/01

(54) **CATHETER ASSEMBLY AND CONVEYING SYSTEM**

(30) Priority: 22.05.2023 CN 202310581999
(71) Applicant: Shanghai MicroPort CardioFlow Medtech Co., Ltd., Shanghai 201203 (CN)
(72) Inventor: YU, Wenxia, Shanghai 201203 (CN); ZHANG, Pinghai, Shanghai 201203 (CN); HUANG, Qingqing, Shanghai 201203 (CN); CHEN, Guoming, Shanghai 201203 (CN)
(74) Representative: Patentship Patentanwaltsgesellschaft mbH
(86) International application number: PCT/CN2024/093327
(87) International publication number: WO 2024/240027

(57) **Abstract**

A catheter assembly and a delivery system. The catheter assembly includes an inner catheter (1000), an inner core tube (2000) and a sealing member (3000). The inner catheter (1000) comprises a catheter lumen axially extending therethrough, and the inner core tube (2000) comprises a core lumen axially extending therethrough and is disposed in the catheter lumen of the inner catheter (1000), and a gap exists between an outer wall of the inner core tube (2000) and an inner wall of the inner catheter (1000). The sealing member (3000) is provided on at least one of the inner catheter (1000) and the inner core tube (2000) to seal the gap between them. The sealing member (3000) serves to seal the gap between the outer wall of the inner core tube (2000) and the inner wall of the inner catheter (1000) before and throughout a procedure so that the sealed gap will not bring the in-vivo environment with communication with the outside world, thereby fundamentally addressing the problem of blood leakage. With the gap being sealed by the sealing member (3000), a challenging air removal operation becomes not necessary any more for a surgeon before the procedure begins, making the procedure overall simpler and faster.

## Description

### TECHNICAL FIELD

The present application relates to the field of medical devices and, in particular, to a catheter assembly and a delivery system.

### BACKGROUND

Heart valve replacement involves using a delivery system to deliver, for example, a mitral, tricuspid, pulmonary or similar valve to a target site, such as the aortic root, and then release the valve there after its correct positioning has been confirmed, replacing the native valve. This has become a highly sought-after, cutting-edge technique in the field of valvular heart disease, marking a milestone in minimally invasive interventional treatment of aortic valve disease. This technique can be used to treat aortic valve disease without thoracotomy, heartbeat stoppage or other highly traumatic intervention as may be necessary for traditional surgical procedures. However, a heart valve replacement procedure using a delivery system may suffer from blood leakage during delivery of a valve by the delivery system, which may impede the performance of the procedure and tends to threaten patient health.

### SUMMARY OF THE INVENTION

In view of the above, it is necessary to provide a catheter assembly and a delivery system, which overcome the above described problem.

Accordingly, there is provided herein a catheter assembly comprising:
an outer sheath comprising a sheath lumen axially extending therethrough;
an inner catheter comprising a catheter lumen axially extending therethrough, wherein the inner catheter is disposed in the sheath lumen of the outer sheath;
an inner core tube comprising a core lumen axially extending therethrough, wherein the inner core tube is disposed in the catheter lumen of the inner catheter, a gap exists between an outer wall of the inner core tube and an inner wall of the inner catheter,
a sealing member that is provided on at least one of the inner catheter and the inner core tube to seal the gap between the inner core tube and the inner catheter; and
a control member configured for connection with the inner catheter to control a bending of the inner catheter, wherein the outer sheath bends along with the inner catheter.

In one embodiment, the inner core tube is movably disposed in the catheter lumen of the inner catheter.

Alternatively, the inner core tube may be fixedly disposed in the catheter lumen of the inner catheter.

In one embodiment, a distal end of the inner core tube protrudes out of a distal end of the inner catheter, wherein the sealing member comprises a first through bore open at both ends, wherein the sealing member is sealingly disposed over the inner core tube though the first through bore, and wherein the sealing member is sealingly coupled to the distal end of the inner catheter.

In one embodiment, the sealing member comprises a first sealing body that sealingly contacts the outer wall of the inner core tube, and wherein the inner core tube is movably disposed in the catheter lumen of the inner catheter.

Alternatively, the sealing member may have a second sealing body that is sealingly attached to the outer wall of the inner core tube, thereby fixedly disposing the inner core tube in the catheter lumen of the inner catheter.

In one embodiment, the first sealing body is an annular sealing collar disposed in the first through bore, wherein the annular sealing collar extends circumferentially around the inner core tube and sealingly contacts the outer wall of the inner core tube.

Alternatively, the second sealing body may be a sealing adhesive adhering to a distal end of the sealing member, and wherein the distal end of the sealing member is sealingly fixed to the outer wall of the inner core tube.

In one embodiment, the catheter assembly comprises:
a securing member comprising a second through bore open at both ends, wherein the securing member is disposed over the inner core tube though the second through bore, the securing member sealingly coupled to the distal end of the inner catheter, wherein the sealing member is indirectly sealingly coupled to the distal end of the inner catheter via the securing member, wherein a third sealing body is disposed between the sealing member and the securing member, wherein the securing member sealingly contacts the outer wall of the inner core tube, and wherein the inner core tube is movably disposed in the catheter lumen of the inner catheter.

In one embodiment, an engagement head portion and an engagement recess are provided at a proximal end of the sealing member and a distal end of the securing member, respectively, wherein the proximal end of the sealing member is sealingly coupled to the distal end of the securing member through the engagement head portion and the engagement recess, wherein the third sealing body is an annular seal extending circumferentially around the inner core tube, sealingly contacts the outer wall of the inner core tube and is located between an end of the engagement head portion and a bottom of the engagement recess.

In one embodiment, the control member is disposed at a proximal end of the inner catheter and coupled to a proximal end of the inner core tube, wherein the control member is connected to the inner catheter through a control wire to control the bending of the inner catheter.

In one embodiment, the catheter assembly comprises:
a distal guide head disposed at a distal end of the inner core tube.

Also provided herein is a delivery system comprising the catheter assembly as defined above.

In the above catheter assembly and delivery system, the sealing member may be provided either on the inner catheter, or on the inner core tube. The sealing member serves to seal the gap between the outer wall of the inner core tube and the inner wall of the inner catheter before and throughout a procedure so that the sealed gap will not bring the in-vivo environment with communication with the outside world, thereby fundamentally addressing the problem of blood leakage. With the gap being sealed by the sealing member, a challenging air removal operation becomes not necessary any more for a surgeon before the procedure begins, making the procedure overall simpler and faster.

### BRIEF DESCRIPTION OF THE DRAWINGS

Fig. 1 schematically illustrates a catheter assembly according to an embodiment of the present application.
Fig. 2 schematically illustrates a catheter assembly according to another embodiment of the present application.
Fig. 3 schematically illustrates a sealing member employing a first sealing approach according to an embodiment of the present application.
Fig. 4 schematically illustrates a sealing member employing a second sealing approach according to an embodiment of the present application.
Fig. 5 is a schematic enlarged view of the sealing member of Fig. 4 that employs the first sealing approach.
Fig. 6 schematically illustrates a sealing member employing a third sealing approach according to an embodiment of the present application.
Fig. 7 schematically illustrates a catheter assembly according to an embodiment of the present application, in which an outer sheath is overshooting.
Fig. 8 schematically illustrates a catheter assembly according to an embodiment of the present application, in which an outer sheath is prevented from overshooting.

### Reference Numerals

A portion without overshooting; B portion with overshooting;
1000 inner catheter; 2000 inner core tube; 3000 sealing member; 4000 securing member; 5000 control member; 6000 control wire; 7000 outer sheath; 8000 distal guide head;
3100 first sealing body; 3200 second sealing body; 3300 third sealing body;
3000a engagement recess; 4000a engagement head portion.

### DETAILED DESCRIPTION

Objects, advantages and features of this application will become more apparent upon reading the following more detailed description of specific embodiments thereof in conjunction with the accompanying drawings. In the following description, numerous details are set forth in order to provide a thorough understanding of the application. However, the present application may be practiced in many other forms than those described herein, and those skilled in the art can make similar improvements without deviating from the spirit of the application. Accordingly, the present application is not limited to the specific embodiments disclosed below.

It will be understood that terms such as "central", "longitudinal", "transverse", "length", "width", "thickness", "upper", "lower", "front", "rear", "left", "right", "vertical", "horizontal", "top", "bottom", "inner", "outer", "clockwise", "counterclockwise", "axial", "radial", "circumferential", etc. may be used herein to describe directional or positional relationships based on the orientations shown in the figures. They are intended merely to facilitate and simplify the explanation of the application and do not indicate or imply that the stated components or elements have to comprise, or be constructed or operated in, particular orientations. Therefore, they are not to be construed as limiting the present application.

In addition, as used herein, the terms "first", "second" and the like are intended only for illustration and are not to be construed as denoting or implying relative importance, or as implicitly indicating the number of the referenced items. Accordingly, defining an item with "first" or "second" is an explicit or implicit indication of the presence of one or at least two such items, unless the context clearly dictates otherwise. As used herein, the term "plurality" means "at least two", such as two or three, unless otherwise clearly defined.

As used herein, unless otherwise clearly specified or defined, the terms "mounted", "coupled", "connected", "secured" and variants thereof should be interpreted in a broad sense. For instance, a connection may be a permanent, detachable or integral connection, or a mechanical or electrical connection, or a direct or indirect connection with one or more intervening media, or an internal communication or interaction between two elements. Those of ordinary skill in the art can understand the specific meanings of the above-mentioned terms herein, depending on their context.

As used herein, unless otherwise clearly specified or defined, when a first feature is described as being "above" or "below" a second feature, it may be either in direct contact with the second feature, or in indirect contact with one or more intervening media being present therebetween. When a first feature is referred to as being "on", "above" or "on top of" a second feature, it may be right or obliquely on, above or on top of the second feature, or simply located at a higher level than the second feature. When a first feature is referred to as being "under", "below" or "at bottom of" a second feature, it may be right or obliquely under, below or at bottom of the second feature, or simply located at a lower level than the second feature.

It should be noted that when a component is referred to as being "secured" to or "disposed" on another component, it may be directly on the other component, or intervening components may be present. When a component is referred to as being "connected" or "coupled" to another component, it can be directly connected or coupled to the other component, or intervening components may be present. As used herein, the terms "vertical", "horizontal", "upper", "lower", "left", "right" and the like are merely illustrative and do not represent the only implementation possible.

To more clearly describe structures of catheter assemblies and delivery systems proposed herein, as used herein, the term "distal end" refers to an end farther away from an operator, and the term "proximal end" refers to an end closer to the operator, during surgical operation. Unless defined otherwise, all technical and scientific terms used herein have the same meaning as commonly understood by one of ordinary skill in the art to which this application belongs. The terminology used herein is for the purpose of describing particular embodiments only and is not intended to be limiting of the application.

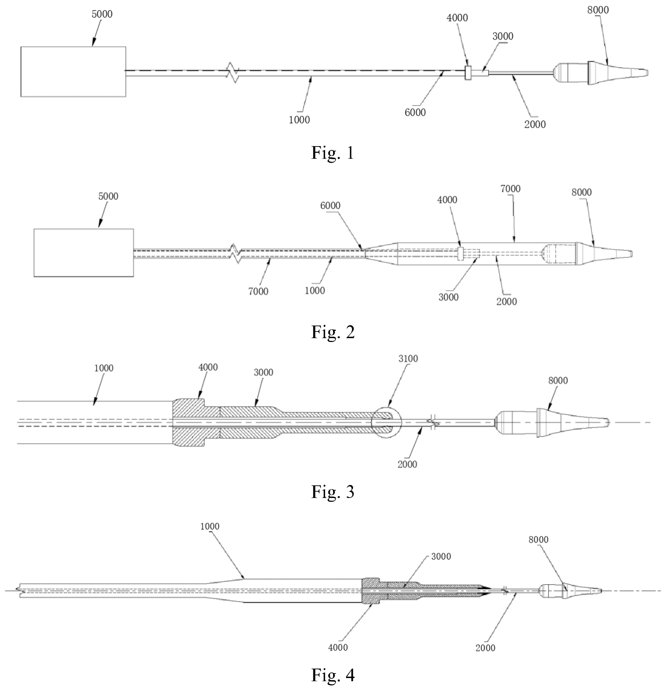

Referring to Fig. 1, in an embodiment described herein, there is provided a catheter assembly including an inner catheter 1000, an inner core tube 2000 and a sealing member 3000. The inner catheter 1000 comprises a catheter lumen axially extending therethrough, and the inner core tube 2000 comprises a core lumen axially extending therethrough. The inner core tube 2000 is disposed in the catheter lumen of the inner catheter 1000, with a gap being left between an outer wall of the inner core tube 2000 and an inner wall of the inner catheter 1000. The sealing member 3000 is provided on at least one of the inner catheter 1000 and the inner core tube 2000 to seal the gap between the inner core tube 2000 and the inner catheter 1000.

The catheter assembly forms part of a delivery system for use in heart valve replacement such as transcatheter aortic valve replacement. Referring to Fig. 2, the catheter assembly may further include an outer sheath 7000, a distal guide head 8000 and a control member 5000. In one embodiment, the outer sheath 7000 comprises a sheath lumen axially extending therethrough, and the inner catheter 1000 is movably disposed in the sheath lumen of the outer sheath 7000. The distal guide head 8000 is disposed at a distal end of the inner core tube 2000 and may be of a tapered or similar shape capable of facilitating advancement of the entire catheter assembly. The control member 5000 is disposed at a proximal end of the inner catheter 1000 and may be fixed therein by various approaches including, for example, without limitation, threaded connection, snap engagement and adhesive bonding. The control member 5000 is used to steer the inner catheter 1000 by various approaches. For example, the control member 5000 is connected to a corresponding position of the inner catheter 1000 through a control wire 6000, the connection may be formed at a location around the middle or a distal end of the inner catheter 1000. The control member 5000 may be manipulated to tighten or loosen the control wire 6000 to bend the inner catheter 1000 in a desired way. Those skilled in the art may select an appropriate configuration, as desired, without departing from the scope of the application.

The presence of the gap between the outer wall of the inner core tube 2000 and the inner wall of the inner catheter 1000 in the catheter assembly requires a surgeon to remove air from the gap, for example, by filling it with a liquid such as physiological saline or a heparin solution, before the delivery system is used for delivery of a valve, because air otherwise present in the gap would tend to bring the in-vivo environment (e.g., a vascular lumen) into communication with the outside world and thus cause blood leakage, which may not only impede the performance of the procedure, but also tends to threaten the patient's health. Further, the presence of the gap would tend to accommodate air therein, which, when entering the in-vivo environment (e.g., a vascular lumen), may also cause certain damage to the patient.

However, limited to a small overall diameter of the delivery system, the inner catheter 1000 and the inner core tube 2000 in the catheter assembly, especially the inner core tube 2000, have to be made with a very small size. Consequently, the gap between the outer wall of the inner core tube 2000 and the inner wall of the inner catheter 1000 is indeed very tiny, making it very challenging to remove air from the gap. In order to overcome this, the proposed catheter assembly additionally includes the sealing member 3000, which may be provided either on the inner catheter 1000, or on the inner core tube 2000, to seal the gap between the outer wall of the inner core tube 2000 and the inner wall of the inner catheter 1000 before and throughout the procedure. The sealed gap will not bring the in-vivo environment with communication with the outside world, thereby fundamentally addressing the problem of blood leakage. With the gap being sealed by the sealing member 3000, the challenging air removal operation becomes not necessary any more for a surgeon before the procedure begins, making the procedure overall simpler and faster.

A distal end of the inner core tube 2000 extends out of the distal end of the inner catheter 1000, and this part is referred to as loading section used to load a valvular stent, so that the valvular stent is located between the inner core tube 2000 and the outer sheath 7000. After being delivered with the catheter assembly to a target site, the valvular stent can be released simply by retracting the outer sheath 7000 and then switch from a crimped configuration to an expanded configuration at the target site. Due to a small diameter of the inner core tube 2000, in the case of the sealing member 3000 being provided on the inner core tube 2000, the valvular stent may be crimped on the sealing member 3000 and hence indirectly loaded over the inner core tube 2000. This facilitates the loading of the valvular stent, on the one hand, and provides support and protection to the inner core tube 2000, because of the valvular stent being supported directly on the sealing member 3000, instead of on the inner core tube 2000, with the sealing member 3000 intervening between the valvular stent and the inner core tube 2000, thereby preventing the inner core tube 2000 from kinking undesirably, for example, during delivery, loading, passage through the aortic arch, release or retrieval of the valvular stent, on the other hand.

In particular, a guide wire may be threaded through the core lumen of the inner core tube 2000 and used to guide the catheter assembly to be advanced during delivery. Specifically, the guide wire may be brought into contact with a wall of a blood vessel, the catheter assembly can follow a path of the guide wire. Moreover, during catheter steering, the guide wire will bend passively in response to bending of the inner catheter 1000 and the inner core tube 2000, avoiding the inner core tube 2000 from kinking undesirably. Thus, the core lumen of the inner core tube 2000 always remains open, advantageously ensuring smooth advancement and passage of the guide wire therethrough. Otherwise, kinking of the inner core tube 2000 may lead to the core lumen being blocked and closed, disallowing passage of the guide wire therethrough. Preventing kinking of the inner core tube 2000 also facilitates robust release of the valvular stent.

With the gap between the inner core tube 2000 and the inner catheter 1000 being sealed with the sealing member 3000, referring to Figs. 3 to 6, the inner core tube 2000 may be movably disposed in the catheter lumen of the inner catheter 1000. Here, by "movably disposed in the catheter lumen of the inner catheter 1000", it is intended to mean that the inner core tube 2000 is at least axially movable relative to the catheter lumen of the inner catheter 1000. In this case, the inner core tube 2000 may be proximally coupled to the control member 5000. Accordingly, the movability of the inner core tube 2000 relative to the inner catheter 1000 would mean that proximal end of the inner core tube remains stationary, while distal end and adjacent portion of the inner core tube is movable, relative to the inner catheter 1000. Alternatively, the inner core tube 2000 may be disposed in the catheter lumen of the inner catheter 1000 in a fixed way, that is, the inner core tube 2000 may be fixedly disposed in the catheter lumen of the inner catheter 1000. In this case, the inner core tube 2000 may not be axially movable at least relative to the catheter lumen of the inner catheter 1000.

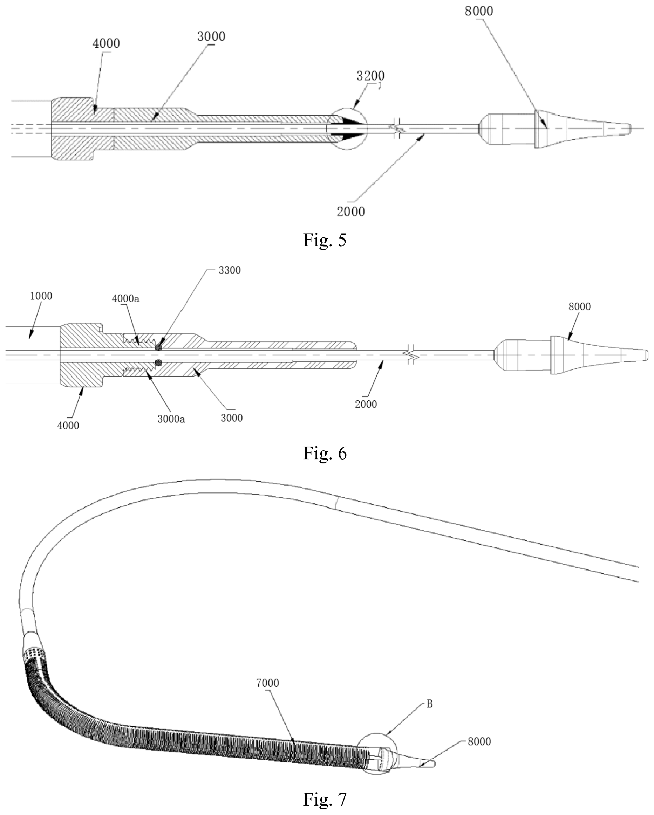

The inner catheter 1000 is usually made of a polymer material, which imparts a certain degree of axial contraction to the inner catheter 1000. Specifically, in a catheter assembly where the inner catheter 1000 is actively steered, the application of steering pulling force causes the inner catheter 1000 to experience a certain degree of axial contraction. Meanwhile, the bending of the outer sheath 7000 is primarily a passive response, induced by the compressive stress from the bent distal end of the inner catheter 1000. In contrast, the outer sheath 7000 itself undergoes no actual axial contraction. Referring to Fig. 7, in some cases, active steering of the catheter assembly may be necessary for its smooth traversing through an aortic arch. Such active steering of the inner catheter 1000 can be accomplished by manipulating the control member 5000 to pull the control wire 6000 and hence a portion of the inner catheter 1000, and which is, for example, located at or around the distal end of the inner catheter 1000. More specifically, as a result of the control wire 6000 being pulled due to the manipulation of the control member 5000, the inner catheter 1000 will be distally bent and actively steered in a desired direction.

However, when the inner catheter 1000 is axially pulled and contracted, relative displacement will occur, in particular axially, between the inner catheter 1000 and the outer sheath 7000. As a consequence, the outer sheath 7000 will move axially distally a distance relative to the inner catheter 1000, which is equal in amount to the axial contraction, eventually leading to a distal end of the outer sheath 7000 "overshooting" the distal end of the inner catheter 1000, as shown in Fig. 7, and interfering with the distal guide head 8000. This would hinder smooth retraction of the outer sheath 7000 together with the delivery system.

With continued reference to Fig. 7, if the inner core tube 2000 is fixed in the catheter lumen of the inner catheter 1000, then when the inner catheter 1000 is pulled by the control wire 6000, the inner core tube 2000 will be contracted along with the inner catheter 1000, leading to a distal end of the outer sheath 7000 coming into interfering contact with the distal guide head 8000 at the distal end of the inner core tube 2000 (because of the "overshooting").

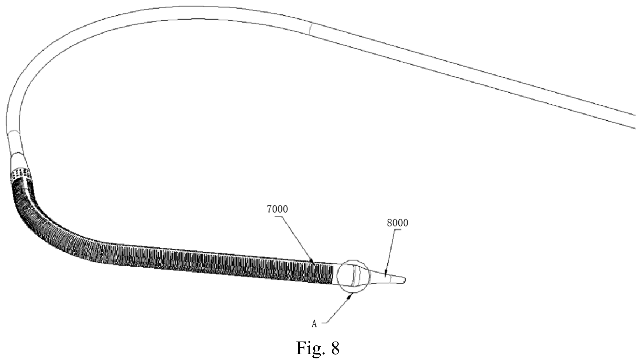

Referring to Fig. 8, if the inner core tube 2000 is movably disposed in the catheter lumen of the inner catheter 1000, it will be able to move axially relative to the catheter lumen of the inner catheter 1000. As such, when the inner catheter 1000 is pulled by the control wire 6000, the inner core tube 2000 may not necessarily be contracted along with the inner catheter 1000. For example, in response to axial contraction of the inner catheter 1000, the inner core tube 2000 may axially move relative to it, but not be axially contracted together with it. As such, the inner core tube 2000 may remain axially stationary relative to the outer sheath 7000, thereby mitigating or eliminating the overshooting of the outer sheath 7000. In particular, when a distal end of the inner core tube 2000 is coupled to the control member 5000, the control member 5000 will resist axial contraction of the inner core tube 2000, maintain it axially stable and unaffected by the axial contraction of the inner catheter 1000.

The sealing member 3000 may be made of a polymer material with relatively low hardness, such as pebax, silicone or the like, by a single injection molding process. The sealing member 3000 may be of any of various suitable structures, as desired. For example, the sealing member 3000 may be of a cylindrical, tapered, stepped or other shape, without departing from the scope of the application.

In one embodiment, the sealing member 3000 may comprise a first through bore open at both ends, which allows the sealing member 3000 to be sealingly disposed over the inner core tube 2000. Meanwhile, the sealing member 3000 may be sealingly coupled to the distal end of the inner catheter 1000. For example, referring to Fig. 3, the sealing member 3000 may have a first sealing body 3100, which sealingly contacts the outer wall of the inner core tube 2000 while not restricting its axial movement. This ensures movability of the inner core tube 2000 within the catheter lumen of the inner catheter 1000. The first sealing body 3100 may be of any of various suitable structures. For example, the first sealing body 3100 may be implemented as an annular sealing collar provided in the first through bore. Specifically, the annular sealing collar may extend circumferentially around the inner core tube 2000, and sealingly contacts its outer wall. Notably, the sealing contact of the annular sealing collar with the outer wall of the inner core tube 2000 does not restrict axial movement of the inner core tube 2000.

The annular sealing collar is also made of a polymer material. As the annular sealing collar is able to seal the gap while not restricting axial movement of the inner core tube 2000, during active steering of the inner catheter 1000, the inner core tube 2000 will not be axially contracted in response to axial contraction of the inner catheter 1000. Therefore, the inner core tube 2000 can remain axially stationary relative to the outer sheath 7000, without causing overshooting of the outer sheath 7000.

Referring to Figs. 4 and 5, in one embodiment, the sealing member 3000 may have a second sealing body 3200 sealingly attached to the outer wall of the inner core tube 2000 so as to seal the gap while restricting axial movement of the inner core tube 2000 relative to the inner catheter 1000, thereby fixing the inner core tube 2000 in the catheter lumen of the inner catheter 1000. The second sealing body 3200 may be of any of various suitable structures. For example, the second sealing body 3200 may be a sealing adhesive, which is adhesived to a distal end of the sealing member 3000 to provide both sealing and attaching effects. The sealing adhesive can sealingly attach the distal end of the sealing member 3000 to the outer wall of the inner core tube 2000, thereby securing the inner core tube 2000 to the inner catheter 1000.

In one embodiment, the catheter assembly may additionally include a securing member 4000 engaging with the lugs or similar structures of the valvular stent to hook the valvular stent. The securing member 4000 may have a groove or similar structure for disposing the control wire 6000. The control wire 6000 is disposed in the groove or similar feature of the securing member 4000 and is connected to and manipulated by the proximal control member 5000. A surgeon can manipulate the control wire 6000 through control member 5000 to actively steer the inner catheter 1000. As shown in Fig. 6, the securing member 4000 may comprise a second through bore open at both ends, which allows the securing member 4000 to be disposed over the inner core tube 2000. At the same time, the securing member 4000 may be sealingly attached to the distal end of the inner catheter 1000. For example, a proximal end of the securing member 4000 may be adhesively bonded to the distal end of the inner catheter 1000 so that the second through bore of the securing member 4000 is brought into communication with the catheter lumen of the inner catheter 1000. Thus, the sealing member 3000 is indirectly sealingly connected to the distal end of the inner catheter 1000 via the securing member 4000.

A third sealing body 3300 may be disposed between the sealing member 3000 and the securing member 4000, the third sealing body 3300 sealingly contacts the outer wall of the inner core tube 2000 while not restricting axial movement of the inner core tube 2000. This ensures movability of the inner core tube 2000 within the catheter lumen of the inner catheter 1000. The third sealing body 3300 may be of any of various suitable structures. For example, the sealing member 3000 may comprise an engagement recess 3000a at its proximal end, and the securing member 4000 may comprise an engagement head portion 4000a at its distal end. In this case, the proximal end of the sealing member 3000 may be sealingly coupled to a distal end of the securing member 4000 by engaging the engagement head portion 4000a in the engagement recess 3000a. The third sealing body 3300 may be an annular seal, which extends circumferentially around the inner core tube 2000, sealingly contacts its outer wall and is located between an end of the engagement head portion 4000a and the bottom of the engagement recess 3000a.

The engagement head portion 4000a may be a plug head portion or threaded head portion, and the engagement recess 3000a may be a socket or threaded recess. For example, the securing member 4000 may be threadedly coupled to the sealing member 3000 by the threaded head portion and the threaded recess. The threaded recess may further comprise an annular sealing groove, and the annular seal may be implemented as a sealing ring made of a polymer material, such as medical silicone, fluorinated rubber or the like. The sealing ring may be disposed on the inner core tube 2000 before the inner core tube 2000 is inserted into the first through bore of the sealing member 3000. The threaded head portion of the securing member 4000 may be then tightened into the threaded recess of the sealing member 3000, thus eventually confining the sealing ring within the annular sealing groove of the sealing member 3000. The continuous compression of the sealing ring by the sealing member 3000 and the securing member 4000 causes it to deform radially, thereby achieving a sealing of the gap between the inner catheter 1000 and the inner core tube 2000, meanwhile it allows the inner core tube 2000 to axially move in the catheter lumen of the inner catheter 1000 to prevent overshooting of the outer sheath 7000.

Also provided herein is a delivery system incorporating the catheter assembly. The structural details, functioning principles and technical benefits of the catheter assembly have been described above and are not repeated again for the sake of brevity. For more information in this regard, reference is made to the above description.

The various technical features of the foregoing embodiments may be combined in any way. Although not all such combinations have been described above for the sake of brevity, any of them is considered to fall within the scope of this specification as long as there is no contradiction between the technical features.

Presented above are merely some embodiments of the present application. Although these embodiments are described with some particularity and in some detail, it should not be construed that they limit the scope of the present application in any sense. It is to be noted that various variations and modifications can be made by those of ordinary skill in the art without departing from the concept of the present application. Accordingly, it is intended that all such variations and modifications are embraced within the scope of this application as defined in the appended claims.

## Claims

1. A catheter assembly, comprising:
an outer sheath comprising a sheath lumen axially extending therethrough;
an inner catheter comprising a catheter lumen axially extending therethrough, wherein the inner catheter is disposed in the sheath lumen of the outer sheath;
an inner core tube comprising a core lumen axially extending therethrough, wherein the inner core tube is disposed in the catheter lumen of the inner catheter, and wherein a gap exists between an outer wall of the inner core tube and an inner wall of the inner catheter,
a sealing member that is provided on at least one of the inner catheter and the inner core tube to seal the gap between the inner core tube and the inner catheter; and
a control member configured for connection with the inner catheter to control a bending of the inner catheter, wherein the outer sheath bends along with the inner catheter.

2. The catheter assembly according to claim 1, wherein the inner core tube is movably disposed in the catheter lumen of the inner catheter, or
wherein the inner core tube is fixedly disposed in the catheter lumen of the inner catheter.

3. The catheter assembly according to claim 1, wherein a distal end of the inner core tube protrudes out of a distal end of the inner catheter, wherein the sealing member comprises a first through bore open at both ends, wherein the sealing member is sealingly disposed over the inner core tube though the first through bore, and wherein the sealing member is sealingly coupled to the distal end of the inner catheter.

4. The catheter assembly according to claim 3, wherein the sealing member comprises a first sealing body that sealingly contacts the outer wall of the inner core tube, and wherein the inner core tube is movably disposed in the catheter lumen of the inner catheter, or
wherein the sealing member has a second sealing body that is sealingly attached to the outer wall of the inner core tube, thereby fixedly disposing the inner core tube in the catheter lumen of the inner catheter.

5. The catheter assembly according to claim 4, wherein the first sealing body is an annular sealing collar disposed in the first through bore, wherein the annular sealing collar extends circumferentially around the inner core tube and sealingly contacts the outer wall of the inner core tube, or
wherein the second sealing body is a sealing adhesive adhering to a distal end of the sealing member, and wherein the distal end of the sealing member is sealingly fixed to the outer wall of the inner core tube through the sealing adhesive.

6. The catheter assembly according to claim 3, comprising:
a securing member comprising a second through bore open at both ends, wherein the securing member is disposed over the inner core tube though the second through bore, wherein the securing member is sealingly coupled to the distal end of the inner catheter, wherein the sealing member is indirectly sealingly coupled to the distal end of the inner catheter via the securing member, wherein a third sealing body is disposed between the sealing member and the securing member, wherein the securing member sealingly contacts the outer wall of the inner core tube, and wherein the inner core tube is movably disposed in the catheter lumen of the inner catheter.

7. The catheter assembly according to claim 6, wherein an engagement head portion and an engagement recess are provided at a proximal end of the sealing member and a distal end of the securing member, respectively, wherein the proximal end of the sealing member is sealingly coupled to the distal end of the securing member through the engagement head portion and the engagement recess, wherein the third sealing body is an annular seal, wherein the annular seal extends circumferentially around the inner core tube, sealingly contacts the outer wall of the inner core tube and is located between an end of the engagement head portion and a bottom of the engagement recess.

8. The catheter assembly according to claim 1, wherein the control member is disposed at a proximal end of the inner catheter and is coupled to a proximal end of the inner core tube, wherein the control member is connected to the inner catheter through a control wire to control the bending of the inner catheter.

9. The catheter assembly according to claim 1, comprising:
a distal guide head disposed at a distal end of the inner core tube.

10. A delivery system, comprising the catheter assembly of any one of claims 1 to 9.
